# EUROPEAN PATENT APPLICATION

(11) **EP 4 617 318 A1**
(43) Date of publication of application: **17.09.2025**
(21) Application number: 23888763.2
(22) Date of filing: 09.11.2023
(51) Int. Cl.: C08L 75/04, A61L 27/34, A61L 29/04, A61L 29/06, A61L 29/08, A61L 31/04, A61L 31/06, A61L 31/10, C08F 20/26, C08G 18/73, C08G 18/75, C08L 51/00, C08L 53/00

(54) **RESIN COMPOSITION AND MOLDED BODY**

(30) Priority: 11.11.2022 JP 2022180712
(71) Applicant: MCPP Innovation LLC, Tokyo 100-8251 (JP); Mitsubishi Chemical Corporation, Tokyo 100-8251 (JP)
(72) Inventor: YOMOGIZAWA, Ayako, Tokyo 100-8251 (JP); OTANI, Go, Tokyo 100-8251 (JP); FUKUTA, Hiroki, Tokyo 100-8251 (JP); TOKURA, Yu, Tokyo 100-8251 (JP)
(74) Representative: Vossius & Partner Patentanwälte Rechtsanwälte mbB
(86) International application number: PCT/JP2023/040387
(87) International publication number: WO 2024/101418

(57) **Abstract**

A resin composition containing: a thermoplastic polyurethane (A1); and a (meth)acrylic-based copolymer (B), wherein the (meth)acrylic-based copolymer (B) is an amphiphilic copolymer, and a difference in a refractive index between the thermoplastic polyurethane (A1) and the (meth)acrylic-based copolymer (B) is 0.018 or less. A resin composition containing: a thermoplastic polyurethane (A2); and a (meth)acrylic-based copolymer (B), wherein the thermoplastic polyurethane (A2) contains a structural unit derived from at least one of an aliphatic isocyanate compound and an alicyclic isocyanate compound, and the (meth)acrylic-based copolymer (B) is an amphiphilic copolymer.

## Description

### Technical Field

The present invention relates to a resin composition and a molded article.

### Background Art

In recent years, in the fields of medical instruments, biochemical analysis, and protein separation and purification, various polymer materials (polystyrene, polypropylene, polyethylene, polyurethane, polyvinyl chloride, polymethyl methacrylate, nylon), glass, and metals such as stainless steel are used for various parts and containers such as various reaction vessels, centrifuge tubes, tubes, syringes, pipettes, filters, and separation columns. However, protein adhesion occurs in all materials, which causes a decrease in detection sensitivity, a decrease in reproducibility, and poor purification.

Catheters, cannulas, stents, plasma separation membranes, and artificial organs such as a heart-lung machine and the like come into contact with circulating blood and metabolites in the body. For this reason, these medical instruments are required to have biocompatibility that suppresses the formation of blood clots caused by protein adhesion, plasma protein adhesion, and the like.

Furthermore, transparency is also required for applications such as cardiovascular catheters including a central venous catheter, a peripheral venous catheter, a coronary artery catheter, and a CV port catheter, a blood collection kit, a medication tube, a regenerative medicine related tube, and the like.

Patent Literature 1 describes a medical tube article molded from a polymer mixture containing a thermoplastic polymer and an amphiphilic block copolymer.

### Citation List

### Paten Literature

Patent Literature 1: JP2013-509218 A

### Summary of Invention

### Technical Problem

With the polymer mixture described in Patent Literature 1, it was difficult to achieve both protein adhesion suppression and transparency.

An object of the present invention is to provide a resin composition and a molded article thereof that are excellent in both protein adhesion suppression ability and transparency.

### Solution to Problem

The present invention has the following gist.

[1] A resin composition comprising:
   a thermoplastic polyurethane (A1); and
   a (meth)acrylic-based copolymer (B),
   wherein the (meth)acrylic-based copolymer (B) is an amphiphilic copolymer, and
   a difference in a refractive index between the thermoplastic polyurethane (A1) and the (meth)acrylic-based copolymer (B) is 0.018 or less, or 0.015 or less.
[2] The resin composition according to [1], wherein the refractive index of the thermoplastic polyurethane (A1) is 1.490 or more and 1.503 or less, or 1.490 or more and 1.500 or less.
[3] The resin composition according to [1] or [2], wherein the refractive index of the (meth)acrylic-based copolymer (B) is 1.472 or more, or 1.475 or more, and 1.521 or less, or 1.518 or less.
[4] A resin composition comprising:
   a thermoplastic polyurethane (A2); and
   a (meth)acrylic-based copolymer (B),
   wherein the thermoplastic polyurethane (A2) comprises a structural unit derived from at least one of an aliphatic isocyanate compound and an alicyclic isocyanate compound, and
   the (meth)acrylic-based copolymer (B) is an amphiphilic copolymer.
[5] The resin composition according to any one of [1] to [3], wherein the thermoplastic polyurethane (A1) comprises a structural unit derived from at least one of an aliphatic isocyanate compound and an alicyclic isocyanate compound.
[6] The resin composition according to [5], wherein the thermoplastic polyurethane (A1) comprises a hard segment comprising a structural unit derived from at least one of the aliphatic isocyanate compound and the alicyclic isocyanate compound and a chain extender, and a soft segment comprising a structural unit derived from a polyol.
[7] The resin composition according to [6], wherein a content of the hard segment is 20% by mass or more and 65% by mass or less based on 100% by mass of the thermoplastic polyurethane (A1).
[8] The resin composition according to [7], wherein the content of the hard segment is 25% by mass or more and 50% by mass or less based on 100% by mass of the thermoplastic polyurethane (A1).
[9] The resin composition according to any one of [1] to [8], wherein a content of the (meth)acrylic-based copolymer (B) is 1 part by mass or more and 50 parts by mass or less based on 100 parts by mass of the resin composition.
[10] The resin composition according to any one of [1] to [9], wherein the (meth)acrylic-based copolymer (B) is an amphiphilic copolymer having a polymer chain (B1) of a monomer unit (b1) represented by the following formula (B1). 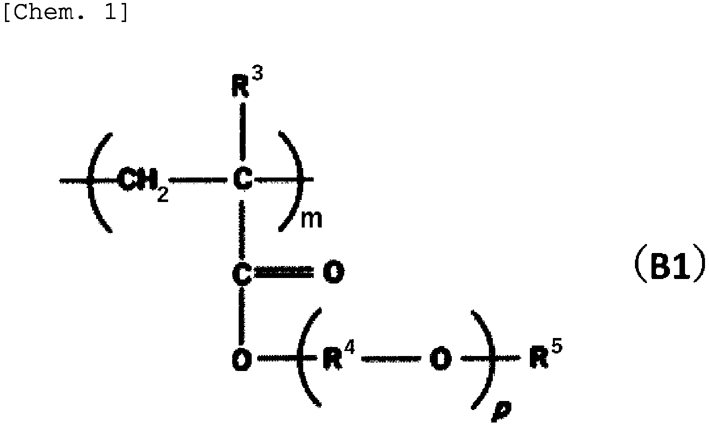 (In the formula (B1), R³ represents a hydrogen atom or a methyl group. R⁴ represents an alkylene group having 1 to 4 carbon atoms. R⁵ represents a hydrocarbon group having 1 to 6 carbon atoms. p is a natural number from 1 to 10. m is a natural number from 2 to 1,000,000.)
[11] The resin composition according to [10], wherein the monomer unit (b1) is a monomer unit derived from at least one monomer (b'1) selected from the group consisting of methoxymethyl acrylate, methoxyethyl acrylate, methoxypropyl acrylate, methoxybutyl acrylate, methoxypolyethylene glycol acrylate, methoxymethyl methacrylate, methoxyethyl methacrylate, methoxypropyl methacrylate, methoxybutyl methacrylate, and methoxypolyethylene glycol methacrylate.
[12] The resin composition according to [10] or [11], wherein the (meth)acrylic-based copolymer (B) is an amphiphilic copolymer having a constituent unit derived from a monomer represented by the following formula (B2). 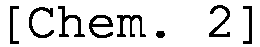 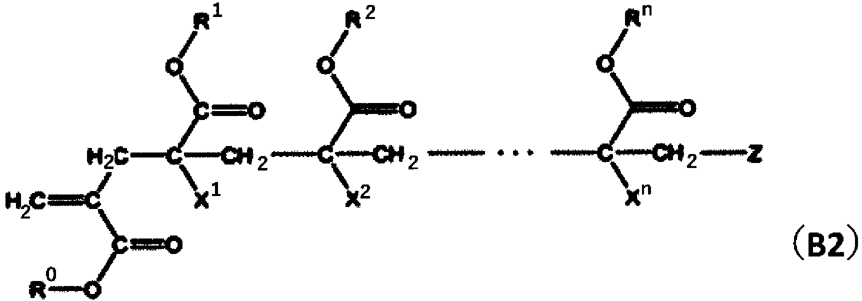 (In the formula (B2), R⁰ to Rⁿ each independently represent a hydrogen atom, an unsubstituted or substituted alkyl group, an unsubstituted or substituted alicyclic group, an unsubstituted or substituted aryl group, an unsubstituted or substituted heteroaryl group, or an unsubstituted or substituted non-aromatic heterocyclic group. Multiple R⁰ to Rⁿ may be the same or different from each other. X¹ to Xⁿ represent a hydrogen atom or a methyl group. Multiple X¹ to Xⁿ may be the same or different from each other. Z is a terminal group. n is a natural number from 2 to 10,000.)
[13] The resin composition according to any one of [1] to [12], wherein the (meth)acrylic-based copolymer (B) has a weight average molecular weight (Mw) of 75,000 or more.
[14] A molding material comprising the resin composition according to any one of [1] to [13].
[15] A medical device part comprising the resin composition according to any one of [1] to [13].
[16] The medical device part according to [15], wherein the medical device part is for in contact with plasma proteins.
[17] The medical device part according to [15] or [16], wherein the medical device part is a tube.
[18] The medical device part according to [15] or [16], wherein the medical device part is a coating.
[19] The medical device part according to [15] or [16], wherein the medical device part is a sealing.
[20] The medical device part according to [15] or [16], wherein the medical device part is a packing.
[21] The medical device part according to [15] or [16], wherein the medical device part is a catheter.

### Advantageous Effects of Invention

The resin composition of the present invention has excellent protein adhesion suppression ability and transparency. Therefore, by using the resin composition of the present invention, it is possible to obtain a molded article suitable for applications requiring antithrombotic properties and transparency, particularly as a medical component.

### Description of Embodiments

Hereinafter, the embodiment of the present invention will be described. The following embodiment is merely an example for explaining the present invention, and is not intended to limit the present invention to only this embodiment. The present invention can be carried out in various forms without departing from the spirit of the invention.

### [Definition of terms]

The following definitions of terms apply throughout the present specification and claims.

"(Meth)acrylic-based monomer" means a monomer having a (meth)acryloyl group.

"(Meth)acryloyl group" is a general term for acryloyl group and methacryloyl group.

"(Meth)acrylate" is a general term for acrylate and methacrylate.

"(Meth)acrylic acid" is a general term for acrylic acid and methacrylic acid.

The use of "to" indicating a range of numerical values means that the numerical values before and after it are included as the lower limit and upper limit.

### <Resin composition>

The resin composition according to the first invention of the present invention is a resin composition comprising: a thermoplastic polyurethane (A1); and a (meth)acrylic-based copolymer (B), wherein the (meth)acrylic-based copolymer (B) is an amphiphilic copolymer, and a difference in a refractive index between the thermoplastic polyurethane (A1) and the (meth)acrylic-based copolymer (B) is 0.018 or less.

The resin composition according to the second invention of the present invention is a resin composition comprising: a thermoplastic polyurethane (A2); and a (meth)acrylic-based copolymer (B), wherein the thermoplastic polyurethane (A2) comprises a structural unit derived from at least one of an aliphatic isocyanate compound and an alicyclic isocyanate compound, and the (meth)acrylic-based copolymer (B) is an amphiphilic copolymer.

Hereinafter, the "first invention" and the "second invention" of the present invention will be collectively referred to as "the present invention".

Furthermore, the "thermoplastic polyurethane (A1)" used in the first invention and the "thermoplastic polyurethane (A2)" used in the second invention will be collectively referred to as the "thermoplastic polyurethane (A) ".

In the first invention, a difference in a refractive index between the thermoplastic polyurethane (A1) and the (meth)acrylic-based copolymer (B) is 0.018 or less, and thus the transparency of the resin composition containing the thermoplastic polyurethane (A1) and the (meth)acrylic-based copolymer (B) is improved.

In the first invention, from the viewpoint of transparency, the difference in the refractive index is more preferably 0.015 or less.

The refractive index of the thermoplastic polyurethane (A1) can be adjusted by changing the composition ratio of an isocyanate compound, a polyol, and the like used in the production of the thermoplastic polyurethane (A1).

The refractive index of the (meth)acrylic-based copolymer (B) can be adjusted by changing the type of monomer constituting the (meth)acrylic-based copolymer (B).

The refractive index is a value measured by a measurement method in accordance with JIS K7142 (Method A).

The transparency of the resin composition can be evaluated by measuring HAZE in accordance with ISO14782.

In the second invention, the thermoplastic polyurethane (A2) contains a structural unit derived from at least one of an aliphatic isocyanate compound and an alicyclic isocyanate compound, thereby improving resistance to yellowing and maintaining transparency for a long period of time.

### <Thermoplastic polyurethane (A1)>

There are no particular limitations on the thermoplastic polyurethane (A1) as long as it is a thermoplastic polymer containing a urethane bond.

The thermoplastic polyurethane (A1) is preferably a polymer consisting of a hard segment including a structural unit derived from an isocyanate compound (a1) having two or more isocyanate groups and a structural unit derived from a chain extender (a2) having two or more active hydroxyl groups at its terminals and a molecular weight of less than 500, and a soft segment including a structural unit derived from a polyol (a3) having two or more active hydrogen groups and a molecular weight of 500 or more.

The isocyanate compound (a1) is not particularly limited, and may be one or a combination of two or more selected from aromatic isocyanate compounds, aliphatic isocyanate compounds, and alicyclic isocyanate compounds.

From the viewpoint of transparency, it is preferable that the thermoplastic polyurethane (A1) is a thermoplastic polyurethane containing a hard segment containing a structural unit derived from at least one of an aliphatic isocyanate compound and an alicyclic isocyanate compound and a chain extender, and a soft segment containing a structural unit derived from a polyol.

The thermoplastic polyurethane (A1) further contains an optional catalyst (a4) and additive (a5) as necessary.

Since the polymerization of the thermoplastic polyurethane (A1) is a polyaddition reaction system, generally, when the content of the hard segment is low, the urethane reaction rate during urethane synthesis tends to be slow. On the other hand, when the content of the hard segment is high, the urethane reaction rate tends to be too fast. From the viewpoint of efficiently carrying out such a polyaddition reaction, the content of the hard segment is preferably 20% by mass or more and 65% by mass or less, and more preferably 25% by mass or more and 65% by mass or less based on 100% by mass of the thermoplastic polyurethane (A1). From the viewpoint of transparency, the content of the hard segment is even more preferably 25% by mass or more and 50% by mass or less based on 100% by mass of the thermoplastic polyurethane (A1).

Examples of the isocyanate compound (a1) include aromatic diisocyanate compounds such as xylylene diisocyanate, 4,4'-diphenyl diisocyanate, 4,4'-diphenylmethane diisocyanate (MDI), tolylene diisocyanate (2,4-tolylene diisocyanate, 2,6-tolylene diisocyanate), o-phenylene diisocyanate, m-phenylene diisocyanate, p-phenylene diisocyanate, 4,4'-diphenyldimethylmethane diisocyanate, 4,4'-dibenzyl diisocyanate, 1,5-naphthylene diisocyanate, 3,3'-dimethyl-4,4'-biphenylene diisocyanate, and m-tetramethylxylylene diisocyanate, and the like; aliphatic diisocyanate compounds such as tetramethylene diisocyanate, 1,5-pentamethylene diisocyanate, 1,6-hexamethylene diisocyanate (HDI), 2,2,4-trimethylhexamethylene diisocyanate, 2,4,4-trimethylhexamethylene diisocyanate, lysine diisocyanate, dimer diisocyanate in which the carboxyl group of a dimer acid is converted to an isocyanate group, and the like; and alicyclic diisocyanate compounds such as 1,4-cyclohexane diisocyanate, isophorone diisocyanate (IPDI), 1-methyl-2,4-cyclohexane diisocyanate, 1-methyl-2,6-cyclohexane diisocyanate, 4,4'-dicyclohexylmethane diisocyanate (H12MDI), 1,3-bis(isocyanatomethyl)cyclohexane, 1,4-bis(isocyanatomethyl)cyclohexane, and the like. These may be used alone or in combination of two or more. From the viewpoint of polymerization stability and mechanical strength, MDI, HDI, and H12MDI are preferred, and H12MDI, which is an alicyclic diisocyanate compound, is particularly preferred.

The chain extender (a2) is a low molecular weight compound having two or more active hydrogen groups at its terminals that react with isocyanate groups, and examples of such compounds include short-chain polyols and polyamines.

Examples of the chain extender (a2) of short-chain polyols include linear diols such as ethylene glycol, 1,3-propanediol, 1,4-butanediol, 1,5-pentanediol, 1,6-hexanediol, 1,8-octanediol, 1,9-nonanediol, 1,10-decanediol, 1,12-dodecanediol, and the like; diols having a branched chain such as propylene glycol, 2-methyl-1,3-propanediol, 2,2-dimethyl-1,3-propanediol, 2,2-diethyl-1,3-propanediol, 2-methyl-2-propyl-1,3-propanediol, 2,4-heptanediol, 1,4-dimethylolhexane, 2-ethyl-1,3-hexanediol, 2,2,4-trimethy- 1,3-pentanediol, 2-methyl-1,8-octanediol, 2-butyl-2-ethyl-1,3-propanediol, dimer diol, and the like; diols having an ether group such as diethylene glycol, dipropylene glycol, triethylene glycol, and the like; diols having an alicyclic structure such as 1,4-cyclohexanediol, 1,4-cyclohexanedimethanol, 1,4-dihydroxyethylcyclohexane, and the like; diols having an aromatic group such as xylylene glycol, 1,4-dihydroxyethylbenzene, 4,4'-methylenebis(hydroxyethylbenzene), and the like; and polyols such as glycerin, trimethylolpropane, pentaerythritol, and the like. These may be used alone or in combination of two or more.

Among these, ethylene glycol, 1,3-propanediol, 1,4-butanediol, and 1,6-hexanediol are particularly preferred.

Examples of the polyamine chain extender (a2) include ethylenediamine, hexamethylenediamine, and isophoronediamine, with ethylenediamine being particularly preferred. These may be used alone or in combination of two or more.

Examples of the polyol (a3) include a polyether polyol, a polycarbonate diol, and a polyester polyol. Among these polyols (a3), a polycarbonate diol is preferred from the viewpoint of long-term durability in vivo.

The number average molecular weight of the polyol (a3) is preferably 500 or more and 5,000 or less, more preferably 900 or more and 3,000 or less, and even more preferably 900 or more and 2,200 or less.

These polyols (a3) may be used alone or in combination of two or more.

When producing the thermoplastic polyurethane (A1), the ratio of the number of moles of the total isocyanate groups in the isocyanate compound (a1) to the number of moles of the total active hydrogen groups in the chain extender (a2) and the polyol (a3) is preferably adjusted to 0.8 to 1.2, and more preferably 0.9 to 1.1.

As a method for producing the thermoplastic polyurethane (A1), a known production method can be applied.

Specific examples include a one-shot method in which the isocyanate compound (a1), the chain extender (a2), and the polyol (a3) are mixed together and reacted; a prepolymer method in which the isocyanate compound (a1) and the polyol (a3) are reacted to prepare a prepolymer having isocyanate groups at both ends, and then the prepolymer is reacted with the chain extender (a2); a solvent polymerization method in which the above reaction is carried out in a solution; and a bulk polymerization method carried out under solvent-free conditions.

When the thermoplastic polyurethane (A1) is produced by a solution polymerization method, one or more of N,N-dimethylformamide, N,N-dimethylacetamide, methyl ethyl ketone, cyclohexanone, ethyl acetate, butyl acetate, and the like can be used as the solvent. Among these, N,N-dimethylacetamide is particularly preferred.

The catalyst (a4) is used to promote the reaction between the isocyanate group of the isocyanate compound (a1) and the active hydrogen groups of the chain extender (a2) and polyol (a3). As the catalyst (a4), known urethane polymerization catalysts such as amine-based catalysts such as triethylamine, N-ethylmorpholine, triethylenediamine, and the like, or tin-based compounds such as stannous octylate, trimethyltin laurate, dibutyltin dilaurate, dioctyltin dilaurate, dioctyltin dineodecanoate, and organometallic salts such as titanium-based compounds can be used.

Examples of the additive (a5) include various additives such as a heat stabilizer, an ultraviolet absorber, a lubricant, an antiblocking agent, and a contrast agent.

Examples of the antiblocking agent include layered silicate minerals such as dry silica and talc.

Examples of the contrast agent include barium sulfate, tungsten, and bismuth, and the like.

These additives (a5) can be added and mixed to the extent that the properties of the thermoplastic polyurethane (A1) are not impaired.

The hardness of the thermoplastic polyurethane (A1) is usually 70A or more and 75D or less.

From the viewpoint of molding process stability, the hardness of the thermoplastic polyurethane (A1) is preferably 72A or more.

The hardness of the thermoplastic polyurethane (A1) is preferably 68D or less. In tube applications such as medical catheters, it is particularly important that the medical catheter is not kinked, so the hardness of the thermoplastic polyurethane (A1) is more preferably 90A or less.

Here, the hardness A and hardness D of the thermoplastic polyurethane (A1) are values measured after 3 seconds according to ISO 7619-1.

In the resin composition of the first invention, the difference between the refractive index of the thermoplastic polyurethane (A1) and the refractive index of the (meth)acrylic-based copolymer (B) described below is 0.018 or less, as described above, and preferably 0.015 or less. From the viewpoint of keeping the difference in the refractive index between the thermoplastic polyurethane (A1) and the (meth)acrylic-based copolymer (B) equal or less than the above upper limit, the refractive index of the thermoplastic polyurethane (A1) is preferably 1.490 or more and 1.503 or less, and more preferably 1.490 or more and 1.500 or less.

The thermoplastic polyurethane (A1) is available as a commercially available product. Examples of commercially available products include the ChronoFlex (registered trademark) AL series manufactured by Mitsubishi Chemical Corporation, the ChronoFlex (registered trademark) C series manufactured by Mitsubishi Chemical Corporation, the ChronoThane (registered trademark) P series manufactured by Mitsubishi Chemical Corporation, the ChronoThane (registered trademark) T series manufactured by Mitsubishi Chemical Corporation, the Carbothane (registered trademark) series manufactured by Lubrizol Corporation, the Bionate (registered trademark) series manufactured by DSM, the Texin (registered trademark) series manufactured by Covestro AG, and the RESAMINE (registered trademark) P series manufactured by Dainichiseika Color & Chemicals Mfg. Co., Ltd. The thermoplastic polyurethane (A1) can be appropriately selected from these commercially available products and used.

Only one type of thermoplastic polyurethane (A1) may be used, or two or more types having different physical properties, copolymer component compositions, structures, and the like may be mixed and used.

### <Thermoplastic polyurethane (A2)>

The thermoplastic polyurethane (A2) contains a structural unit derived from at least one of an aliphatic isocyanate compound and an alicyclic isocyanate compound, and is preferably a polymer consisting of a hard segment containing the structural unit derived from at least one of the aliphatic isocyanate compound and the alicyclic isocyanate compound and a structural unit derived from the chain extender (a2) having two or more active hydroxyl groups at its terminal and a molecular weight of less than 500, and a soft segment containing a structural unit derived from the polyol (a3) having two or more active hydrogen groups and a molecular weight of 500 or more.

The thermoplastic polyurethane (A2) further contains an optional catalyst (a4) and additive (a5) as necessary.

Examples of the aliphatic isocyanate compound and the alicyclic isocyanate compound include the aforementioned aliphatic diisocyanate compounds such as tetramethylene diisocyanate, 1,5-pentamethylene diisocyanate, 1,6-hexamethylene diisocyanate (HDI), 2,2,4-trimethylhexamethylene diisocyanate, 2,4,4-trimethylhexamethylene diisocyanate, lysine diisocyanate, dimer diisocyanate in which the carboxyl group of a dimer acid is converted to an isocyanate group, and the like; and the aforementioned alicyclic diisocyanate compounds such as 1,4-cyclohexane diisocyanate, isophorone diisocyanate (IPDI), 1-methyl-2,4-cyclohexane diisocyanate, 1-methyl-2,6-cyclohexane diisocyanate, 4,4'-dicyclohexylmethane diisocyanate (H12MDI), 1,3-bis(isocyanatomethyl)cyclohexane, 1,4-bis(isocyanatomethyl)cyclohexane, and the like. These may be used alone or in combination of two or more. From the viewpoint of polymerization stability and mechanical strength, MDI, HDI, and H12MDI are preferred, and H12MDI, which is an alicyclic diisocyanate compound, is particularly preferred.

The chain extender (a2), polyol (a3), catalyst (a4), and additive (a5) can be the same as those used for the aforementioned thermoplastic polyurethane (A1).

The thermoplastic polyurethane (A2) can be produced in the same manner as the aforementioned thermoplastic polyurethane (A1).

The thermoplastic polyurethane (A2) is available as a commercially available product. Examples of commercially available products include the ChronoFlex (registered trademark) AL series manufactured by Mitsubishi Chemical Corporation, the ChronoThane (registered trademark) T series manufactured by Mitsubishi Chemical Corporation, and the Carbothane (registered trademark) series manufactured by Lubrizol Corporation. The thermoplastic polyurethane (A2) can be appropriately selected from these commercially available products and used.

Only one type of thermoplastic polyurethane (A2) may be used, or two or more types having different physical properties, copolymer component compositions, structures, and the like may be mixed and used.

### <(Meth) (meth)acrylic-based copolymer (B)>

The (meth)acrylic-based copolymer (B) is an amphiphilic copolymer.

The resin composition of the present invention can suppress protein adhesion by including an amphiphilic (meth)acrylic-based copolymer (B).

The amphiphilic copolymer has a hydrophilic polymer chain consisting of hydrophilic monomer units and a hydrophobic polymer chain consisting of hydrophobic monomer units.

Examples of the hydrophilic monomer include known hydrophilic monomers such as hydroxyl group-containing (meth)acrylic acid esters, amino group-containing (meth)acrylic acid ester-based vinyl-based monomers, amide group-containing vinyl-based monomers, glycol ester-based monomers, and the like.

Examples of the hydrophilic monomer include the following.

Hydroxyl group-containing (meth)acrylic acid esters such as 2-hydroxyethyl (meth)acrylate, 2-hydroxypropyl (meth)acrylate, 2-hydroxybutyl (meth)acrylate, 3-hydroxybutyl (meth)acrylate, 4-hydroxybutyl (meth)acrylate, glycerol (meth)acrylate, and the like.

Amino group-containing (meth)acrylic acid ester-based vinyl monomers such as dimethylaminoethyl (meth)acrylate, diethylaminoethyl (meth)acrylate, and the like.

Amido group-containing vinyl monomers such as (meth)acrylamide, dimethyl (meth)acrylamide, diethyl (meth)acrylamide, N-tert-butyl (meth)acrylamide, N-methylol (meth)acrylamide, N-isopropylacrylamide, hydroxyethyl acrylamide, N-methoxymethyl (meth)acrylamide, N-butoxymethyl (meth)acrylamide, diacetone acrylamide, maleic acid amide, maleimide, and the like.

Glycol ester-based monomers such as polyethylene glycol (meth)acrylate, polypropylene glycol (meth)acrylate, methoxyethyl (meth)acrylate, ethoxyethyl (meth)acrylate, n-butoxyethyl (meth)acrylate, isobutoxyethyl (meth)acrylate, t-butoxyethyl (meth)acrylate, ethoxyethoxyethyl (meth)acrylate, phenoxyethyl (meth)acrylate, nonylphenoxyethyl (meth)acrylate, 3-methoxybutyl (meth)acrylate, acetoxyethyl (meth)acrylate, "Placcel FM" (caprolactone addition monomer, product name, manufactured by Daicel Chemical Industries, Ltd.), "Blemmer (registered trademark) PME-100" (methoxypolyethylene glycol methacrylate (having two ethylene glycol chains), product name, manufactured by NOF Corporation), "Blemmer (registered trademark) PME-200" (methoxypolyethylene glycol methacrylate (having four ethylene glycol chains), product name, manufactured by NOF Corporation), "BLEMMER (registered trademark) PME-400" (methoxypolyethylene glycol methacrylate (having nine ethylene glycol chains), product name, manufactured by NOF Corporation), "BLEMMER (registered trademark) 50POEP-800B" (octoxypolyethylene glycol-polypropylene glycol-methacrylate (having eight ethylene glycol chains and six propylene glycol chains), product name, manufactured by NOF Corporation), "BLEMMER (registered trademark) 20ANEP-600" (nonylphenoxy (ethylene glycol-polypropylene glycol) monoacrylate, product name, manufactured by NOF Corporation), "BLEMMER (registered trademark) AME-100" (product name, manufactured by NOF Corporation), "BLEMMER (registered trademark) AME-200" (product name, manufactured by NOF Corporation), "BLEMMER (registered trademark) 50AOEP-800B" (product name, manufactured by NOF Corporation), and the like.

These may be used alone or in combination of two or more.

The hydrophobic monomer has a lower solubility in water than the hydrophilic monomer.

Examples of the hydrophobic monomer include the following.

Hydrocarbon group-containing (meth)acrylic acid esters such as methyl (meth)acrylate, ethyl (meth)acrylate, n-propyl (meth)acrylate, isopropyl (meth)acrylate, n-butyl (meth)acrylate, isobutyl (meth)acrylate, tert-butyl (meth)acrylate, isoamyl (meth)acrylate, hexyl (meth)acrylate, 2-ethylhexyl (meth)acrylate, octyl (meth)acrylate, isooctyl (meth)acrylate, nonyl (meth)acrylate, isononyl (meth)acrylate, decyl (meth)acrylate, isodecyl (meth)acrylate, lauryl (meth)acrylate, hexadecyl (meth)acrylate, stearyl (meth)acrylate, isostearyl (meth)acrylate, phenyl (meth)acrylate, benzyl (meth)acrylate, cyclohexyl (meth)acrylate, tetrahydrofurfuryl (meth)acrylate, isobornyl (meth)acrylate, 3,5,5-trimethylcyclohexyl (meth)acrylate, dicyclopentanyl (meth)acrylate, dicyclopentenyl (meth)acrylate, dicyclopentenyloxyethyl (meth)acrylate, terpene acrylate and derivatives thereof, hydrogenated rosin acrylate and derivatives thereof, docosyl (meth)acrylate, and the like.

Epoxy group-containing vinyl-based monomers such as glycidyl (meth)acrylate, glycidyl α-ethyl acrylate, 3,4-epoxybutyl (meth)acrylate, and the like.

Monomers containing a silane coupling agent such as 3-(meth)acryloxypropyltrimethoxysilane, 3-(meth)acryloxypropylmethyldiethoxysilane, 3-(meth)acryloxypropyltriethoxysilane, 3-acryloxypropyltrimethoxysilane, vinyltrimethoxysilane, vinyltriethoxysilane, and the like; organosilyl group-containing monomers other than silane coupling agent-containing monomers, such as trimethylsilyl (meth)acrylate, triethylsilyl (meth)acrylate, tri-n-propylsilyl (meth)acrylate, tri-n-butylsilyl (meth)acrylate, tri-n-amylsilyl (meth)acrylate, tri-n-hexylsilyl (meth)acrylate, tri-n-octylsilyl (meth)acrylate, tri-n-dodecylsilyl (meth)acrylate, triphenylsilyl (meth)acrylate, tri-p-methylphenylsilyl (meth)acrylate, tribenzylsilyl (meth)acrylate, triisopropylsilyl (meth)acrylate, triisobutylsilyl (meth)acrylate, tri-s-butylsilyl (meth)acrylate, tri-2-methylisopropylsilyl (meth)acrylate, tri-tert-butylsilyl (meth)acrylate, ethyldimethylsilyl (meth)acrylate, n-butyldimethylsilyl (meth)acrylate, diisopropyl-n-butylsilyl (meth)acrylate, n-octyldi-n-butylsilyl (meth)acrylate, diisopropylstearylsilyl (meth)acrylate, dicyclohexyl (meth)acrylate, tert-butyldiphenylsilyl (meth)acrylate, lauryldiphenylsilyl (meth)acrylate, triisopropylsilyl methyl maleate, triisopropylsilyl amyl maleate, tri-n-butylsilyl-n-butyl maleate, tert-butyldiphenylsilyl methyl maleate, tert-butyldiphenylsilyl-n-butyl maleate, triisopropylsilyl methyl fumarate, triisopropylsilyl amyl fumarate, tri-n-butylsilyl-n-butyl fumarate, tert-butyldiphenylsilyl methyl fumarate, tert-butyldiphenylsilyl-n-butyl fumarate, Silaplane (registered trademark) FM-0711 (manufactured by JNC Corporation, product name), Silaplane (registered trademark) FM-0721 (manufactured by JNC Corporation, product name), Silaplane (registered trademark) FM-0725 (manufactured by JNC Corporation, product name), Silaplane (registered trademark) TM-0701 (manufactured by JNC Corporation, product name), Silaplane (registered trademark) TM-0701T (manufactured by JNC Corporation, product name), X-22-174ASX (manufactured by Shin-Etsu Chemical Co., Ltd., product name), X-22-174BX (manufactured by Shin-Etsu Chemical Co., Ltd., product name), KF-2012 (manufactured by Shin-Etsu Chemical Co., Ltd., product name), X-22-2426 (manufactured by Shin-Etsu Chemical Co., Ltd., product name), X-22-2404 (manufactured by Shin-Etsu Chemical Co., Ltd., product name), and the like.

These may be used alone or in combination of two or more.

As the hydrophilic polymer chain, for example, a polymer chain (B1) of a monomer unit (b1) represented by the following formula (B1) is preferred. 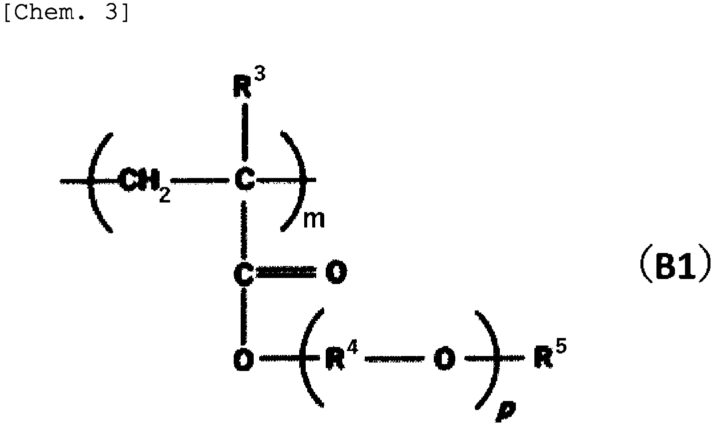 (In the formula (B1), R³ represents a hydrogen atom or a methyl group. R⁴ represents an alkylene group having 1 to 4 carbon atoms. R⁵ represents a hydrocarbon group having 1 to 6 carbon atoms. p is a natural number from 1 to 10. m is a natural number from 2 to 1,000,000.)

The monomer unit (b1) is a monomer unit derived from a monomer (b'1).

Examples of the monomer (b'1) include methoxymethyl acrylate, methoxyethyl acrylate, methoxypropyl acrylate, methoxybutyl acrylate, ethoxymethyl acrylate, ethoxyethyl acrylate, ethoxypropyl acrylate, ethoxybutyl acrylate, propoxymethyl acrylate, propoxyethyl acrylate, propoxypropyl acrylate, propoxybutyl acrylate, butoxymethyl acrylate, butoxyethyl acrylate, butoxypropyl acrylate, butoxybutyl acrylate, methoxymethyl methacrylate, methoxyethyl methacrylate, methoxypropyl methacrylate, methoxybutyl methacrylate, ethoxymethyl methacrylate, ethoxyethyl methacrylate, ethoxypropyl methacrylate, ethoxybutyl methacrylate, propoxymethyl methacrylate, propoxyethyl methacrylate, propoxypropyl methacrylate, propoxybutyl methacrylate, butoxymethyl methacrylate, butoxyethyl methacrylate, butoxypropyl methacrylate, butoxybutyl methacrylate, methoxypolyethylene glycol acrylate, methoxypolyethylene glycol methacrylate, and the like. Examples of the commercially available methoxypolyethylene glycol methacrylates include "Blemmer (registered trademark) PME-100" (methoxypolyethylene glycol methacrylate (having 2 ethylene glycol chains) manufactured by NOF Corporation, product name), "Blemmer (registered trademark) PME-200" (methoxypolyethylene glycol methacrylate (having 4 ethylene glycol chains) manufactured by NOF Corporation, product name), and the like. These may be used alone or in combination of two or more.

Among these, as the monomer (b'1), from the viewpoint of suppressing protein adhesion, methoxymethyl acrylate, methoxyethyl acrylate, methoxypropyl acrylate, methoxybutyl acrylate, methoxypolyethylene glycol acrylate, methoxymethyl methacrylate, methoxyethyl methacrylate, methoxypropyl methacrylate, methoxybutyl methacrylate, and methoxypolyethylene glycol methacrylate are preferred, methoxyethyl acrylate, methoxypropyl acrylate, methoxypolyethylene glycol acrylate, and methoxypolyethylene glycol methacrylate are more preferred, and methoxyethyl acrylate and methoxyethyl methacrylate are particularly preferred.

The degree of polymerization m of the monomer unit (b1) in the polymer chain (B1) is a natural number of 2 to 1,000,000, however from the viewpoint of suppressing protein adhesion, a natural number of 2 to 100,000 is preferred, and a natural number of 5 to 50,000 is even more preferred. When the degree of polymerization m is 2 or more, the protein adhesion suppression effect is more excellent. When the degree of polymerization m is 1,000,000 or less, the moldability is more excellent.

The content of the monomer unit (b1) is preferably 10% by mass or more, more preferably 20% by mass or more, even more preferably 30% by mass or more, and particularly preferably 40% by mass or more, based on 100% by mass of the total of the monomer units present in the (meth)acrylic-based copolymer (B). The content of the monomer unit (b1) is preferably 90% by mass or less, more preferably 80% by mass or less, even more preferably 70% by mass or less, and particularly preferably 60% by mass or less. When the content of the monomer unit (b1) is 10% by mass or more, the protein adhesion suppression effect is excellent. When the content of the monomer unit (b1) is 90% by mass or less, the moldability is excellent.

As the hydrophobic polymer chain, for example, a polymer chain having a constituent unit derived from a monomer represented by the following formula (B2) is preferable. 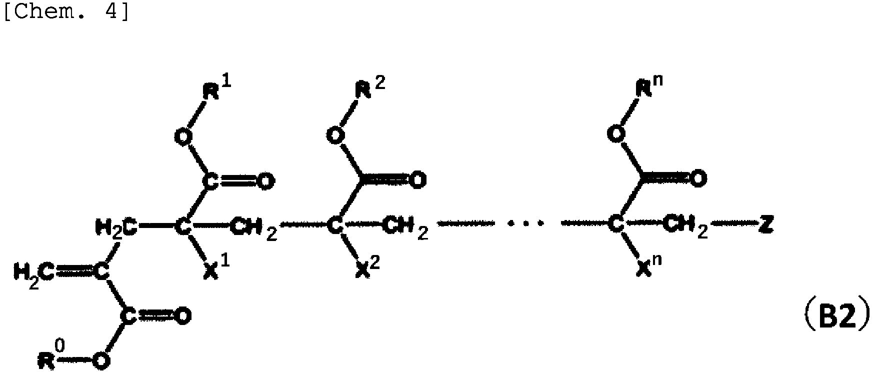 (In the formula (B2), R⁰ to Rⁿ each independently represent a hydrogen atom, an unsubstituted or substituted alkyl group, an unsubstituted or substituted alicyclic group, an unsubstituted or substituted aryl group, an unsubstituted or substituted heteroaryl group, or an unsubstituted or substituted non-aromatic heterocyclic group. Multiple R⁰ to Rⁿ may be the same or different from each other. X¹ to Xⁿ represent a hydrogen atom or a methyl group. Multiple X¹ to Xⁿ may be the same or different from each other. Z is a terminal group. n is a natural number from 2 to 10,000.)

In the formula (B2), R⁰ to Rⁿ each independently represent a hydrogen atom, an unsubstituted or substituted alkyl group, an unsubstituted or substituted alicyclic group, an unsubstituted or substituted aryl group, an unsubstituted or substituted heteroaryl group, or an unsubstituted or substituted non-aromatic heterocyclic group. Multiple R⁰ to Rⁿ may be the same or different from each other.

From the viewpoint of maintaining hydrophobicity, R⁰ to Rⁿ each independently are preferably an alkyl group, an alicyclic group, an aryl group, a heteroaryl group, or a non-aromatic heterocyclic group, more preferably an alkyl group or an alicyclic group, and particularly preferably an alkyl group.

From the viewpoint of availability, as the alkyl groups of the R^{Y} to Rⁿ methyl group, an ethyl group, a **n-**propyl group, an i-propyl group, a n-butyl group, a t-butyl group, a pentyl group, a hexyl group, a heptyl group, an octyl group, a cyclopropyl group, a cyclobutyl group, an isobornyl group, and an adamantly group are preferred, and a methyl group, an ethyl group, a n-propyl group, an i-propyl group, a n-butyl group, a t-butyl group, a cyclopropyl group, a cyclobutyl group, an isobornyl group, and an adamantyl group are more preferred.

X¹ to Xⁿ represent a hydrogen atom or a methyl group. Multiple X¹ to Xⁿ may be the same or different from each other.

Z is a terminal group. Examples of the terminal group include a hydrogen atom, a group derived from a radical polymerization initiator, a radically polymerizable group, and the like, as in the case of a terminal group of a polymer obtained by known radical polymerization.

In the formula (B2), n is a natural number from 2 to 10,000. From the viewpoint of moldability, n is preferably 2 to 1,000, more preferably 5 to 1,000, even more preferably 10 to 500, and particularly preferably 20 to 500.

The number average molecular weight (Mn) of the monomer represented by the formula (B2) is preferably 200 to 100,000, more preferably 500 to 100,000, even more preferably 1,000 to 50,000, and particularly preferably 2,000 to 50,000. When the number average molecular weight is 200 to 100,000, the moldability is more excellent.

The number average molecular weight is calculated as a relative molecular weight by gel permeation chromatography (GPC) using polymethyl methacrylate as a standard resin. The specific measurement method is as described in the Examples section below.

The content of the constituent unit derived from the monomer represented by the formula (B2) is preferably 10% by mass or more, more preferably 30% by mass or more, and even more preferably 40% by mass or more, based on 100% by mass of the total of the monomer units present in the (meth)acrylic-based copolymer (B). The content of the constituent unit derived from the monomer represented by the formula (B2) is preferably 90% by mass or less, more preferably 80% by mass or less, and even more preferably 70% by mass or less. When the content of the constituent unit derived from the monomer represented by the formula (B2) is 20% by mass or more, the moldability is excellent. When the content of the constituent units derived from the monomer represented by the formula (B2) is 90% by mass or less, the protein adhesion suppression effect is excellent.

The (meth)acrylic-based copolymer (B) may contain a constituent unit derived from a monomer (b2) other than the polymer chain (B1) of the monomer unit (b1) represented by the formula (B1) and the constituent unit derived from the monomer represented by the formula (B2).

The monomer (b2) may be any monomer capable of copolymerizing with the monomer (b'1) and the monomer represented by the formula (B2).

Examples of the monomer (b2) include the following.

Vinyl-based monomers such as styrene, α-methylstyrene, vinyl toluene, (meth)acrylonitrile, vinyl chloride, vinyl acetate, vinyl propionate, and the like.

Polyfunctional vinyl monomers such as divinylbenzene, ethylene glycol di(meth)acrylate, 1,3-butylene glycol di(meth)acrylate, 1,4-butanediol di(meth)acrylate, 1,6-hexanediol di(meth)acrylate, diethylene glycol di(meth)acrylate, triethylene glycol di(meth)acrylate, tetraethylene glycol di(meth)acrylate, polyethylene glycol di(meth)acrylate, tripropylene glycol di(meth)acrylate, neopentyl glycol di(meth)acrylate, 1,9-nonanediol di(meth)acrylate, 1,10-decanediol di(meth)acrylate, trimethylolpropane tri(meth)acrylate, pentaerythritol tetra(meth)acrylate, dipentaerythritol hexa(meth)acrylate, allyl (meth)acrylate, triallyl cyanurate, diallyl maleate, polypropylene glycol diallyl ether, N,N'-methylene bis(meth)acrylamide, and the like.

Heterocyclic- ased monomers such as (meth)acryloylmorpholine, vinylpyrrolidone, vinylpyridine, vinylcarbazole, and the like.

Halogenated olefins such as vinyl chloride, vinylidene chloride, vinyl fluoride, vinylidene fluoride, chlorotrifluoroethylene, and the like.

2-Isocyanatoethyl (meth)acrylate.

Fluorine-containing monomers (excluding halogenated olefins) such as 2,2,2-trifluoroethyl (meth)acrylate, 2,2,3,3,3-pentafluorophenyl (meth)acrylate, 2-(perfluorobutyl)ethyl (meth)acrylate, 3-(perfluorobutyl)-2-hydroxypropyl (meth)acrylate, 2-(perfluorohexyl)ethyl (meth)acrylate, 3-perfluorohexyl-2-hydroxypropyl (meth)acrylate, 3-(perfluoro-3-methylbutyl)-2-hydroxypropyl (meth)acrylate, 2,2,3,3-tetrafluoropropyl (meth)acrylate, 1H,1H,5H-octafluoropentyl (meth)acrylate, 1H,1H,5H-octafluoropentyl (meth)methacrylate, 1H,1H,2H,2H-tridecafluorooctyl (meth)acrylate, 1H-1-(trifluoromethyl)trifluoroethyl (meth)acrylate, 1H,1H,3H-hexafluorobutyl (meth)acrylate, 1,2,2,2-tetrafluoro-1-(trifluoromethyl)ethyl (meth)acrylate, and the like.

Carboxyl group-containing vinyl-based monomers such as 2-(meth)acryloyloxyethyl hexahydrophthalic acid, 2-(meth)acryloyloxypropyl hexahydrophthalic acid, 2-(meth)acryloyloxyethyl phthalic acid, 2-(meth)acryloyloxypropyl phthalic acid, 2-(meth)acryloyloxyethyl maleic acid, 2-(meth)acryloyloxypropyl maleic acid, 2-(meth)acryloyloxyethyl succinic acid, 2-(meth)acryloyloxypropyl succinic acid, (meth)acrylic acid, crotonic acid, fumaric acid, maleic acid, itaconic acid, citraconic acid, monomethyl maleate, monoethyl maleate, monooctyl maleate, monomethyl itaconate, monoethyl itaconate, monobutyl itaconate, monooctyl itaconate, monomethyl fumarate, monoethyl fumarate, monobutyl fumarate, monooctyl fumarate, monoethyl citraconate, and the like.

Acid anhydride group-containing vinyl-based monomers such as maleic anhydride, itaconic anhydride, and the like.

Unsaturated dicarboxylic acid diester monomers such as dimethyl maleate, dibutyl maleate, dimethyl fumarate, dibutyl fumarate, dibutyl itaconate, diperfluorocyclohexyl fumarate, and the like.

Heterocyclic-based monomers such as (meth)acryloylmorpholine, vinylpyrrolidone, vinylpyridine, vinylcarbazole, and the like.

These may be used alone or in combination of two or more.

When the (meth)acrylic-based copolymer (B) contains the constituent unit derived from the monomer (b2), the content of the constituent unit derived from the monomer (b2) is preferably 0 to 33% by mass, more preferably 0 to 25% by mass, and even more preferably 0.5 to 20% by mass and may be 0% by mass, based on 100% by mass of the total of the monomer units present in the (meth)acrylic-based copolymer (B). When the content of the constituent unit derived from the monomer (b2) is 33% by mass or less, the functions of the polymer chain represented by the formula (B1) and the constituent unit derived from the monomer represented by the formula (B2) are not impaired.

The mass average molecular weight (Mw) of the (meth)acrylic-based copolymer (B) is preferably 75,000 or more, more preferably 75,000 or more and 1,000,000 or less, and even more preferably 80,000 or more and 500,000 or less. When the mass average molecular weight is 75,000 or more and 1,000,000 or less, the moldability is excellent.

Here, the mass average molecular weight of the (meth)acrylic-based copolymer (B) is calculated as a relative molecular weight by gel permeation chromatography (GPC) using polymethyl methacrylate as a standard resin. A specific measurement method is as described in the Examples section below.

The (meth)acrylic-based copolymer (B) preferably has at least one of the structures of a block copolymer and a graft copolymer having the polymer chain (B1) represented by the formula (B1) and the constituent unit derived from the monomer represented by the formula (B2).

In the (meth)acrylic-based copolymer (B), the polymer chain (B1) represented by the formula (B1) has the main effect of suppressing protein adhesion. In the (meth)acrylic-based copolymer (B), the constituent unit derived from the monomer represented by the formula (B2) has the effect of imparting miscibility and compatibility with the thermoplastic polyurethane (A1) and the effect of enabling the (meth)acrylic-based copolymer (B) to be handled as a solid.

At least one of the block and graft structures possessed by the (meth)acrylic-based copolymer (B) may be any of diblock, triblock, multiblock, graft, cyclic, star, comb, dendritic, ladder, and the like, or may be a combination of a plurality of these structures. Among these structures, it is preferable to have at least one of the diblock, triblock, and graft structures, since it is expected to impart the ability to inhibit protein adhesion to the surface of the molded article and is relatively easy to manufacture.

In the resin composition of the first invention, the difference between the refractive index of the thermoplastic polyurethane (A1) and the refractive index of the (meth)acrylic-based copolymer (B) is 0.018 or less, preferably 0.015 or less, as described above. From the viewpoint of making the difference between the refractive index of the thermoplastic polyurethane (A1) and the (meth)acrylic-based copolymer (B) equal to or less than the upper limit, the refractive index of the (meth)acrylic-based copolymer (B) is preferably 1.472 or more, more preferably 1.475 or more. On the other hand, it is preferably 1.521 or less, and more preferably 1.518 or less.

The content of the (meth)acrylic-based copolymer (B) in 100 parts by mass of the resin composition of the present invention is preferably 1 part by mass or more, more preferably 5 parts by mass or more, and even more preferably 10 parts by mass or more. The content of the (meth)acrylic-based copolymer (B) in 100 parts by mass of the resin composition of the present invention is preferably 50 parts by mass or less, more preferably 30 parts by mass or less, and even more preferably 20 parts by mass or less. When the content of (meth)acrylic-based copolymer (B) is 1 part by mass or more and 50 parts by mass or less, the protein adhesion suppression ability and moldability are excellent.

In particular, from the viewpoint of suppressing protein adhesion, the content of (meth)acrylic-based copolymer (B) in the resin composition of the present invention is preferably 10% by mass or more, and more preferably 20% by mass or more. Also, from the viewpoint of breaking strength, it is preferably 50% by mass or less, and more preferably 35% by mass or less.

### <Method of producing the (meth)acrylic-based copolymer (B)>

The (meth)acrylic-based copolymer (B) can be produced by a known polymerization method. Examples of the method for producing the (meth)acrylic-based copolymer (B) include a living polymerization method and a method using a macromonomer.

Examples of the living polymerization method include a living radical polymerization method, a living anionic polymerization method, and the like. Examples of the living radical polymerization methods include reversible addition-fragmentation chain transfer polymerization (RAFT), atom transfer radical polymerization (ATRP), nitroxide-mediated polymerization (NMP), living radical polymerization with organotellurium as a growth terminal (TERP), and the like.

In the present invention, the method of using the monomer represented by the above mentioned formula (B2) as the macromonomer is preferable since it has an advantage in that the (meth)acrylic-based copolymer (B) can be produced relatively easily and has the advantage that it does not require the steps of removing the residues of catalysts and auxiliaries and terminal treatment process, which are necessary in the living polymerization method.

The macromonomer, i.e., the monomer represented by the formula (B2) can be produced by a known method.

Examples of methods for producing the macromonomer include a method using a cobalt chain transfer agent (U.S. 4,680,352 B), a method using an α-substituted unsaturated compound such as α-bromomethylstyrene and the like as a chain transfer agent (WO 88/04304 A), a method of chemically bonding a polymerizable group (JP S60-133007 B, U.S. 5,147,952 B, and JP H06-298921 A), a method using thermal decomposition (JP H11-240854 A), and the like.

Among these, the method using a cobalt chain transfer agent is preferred as the method for producing the macromonomer, since it requires fewer production steps and uses a catalyst having a high chain transfer constant.

To produce the (meth)acrylic-based copolymer (B) using the macromonomer, a monomer mixture containing the macromonomer may be bulk-polymerized, suspension-polymerized, or solution-polymerized by a known method.

### <Other polymers>

The resin composition of the present invention may contain a (meth)acrylic polymer other than the (meth)acrylic-based copolymer (B), for example, a (meth)acrylic polymer of an anti-blocking particles (Q) described below, but the content is preferably 20% by mass or less in the resin composition.

When the content of the (meth)acrylic polymer other than the (meth)acrylic-based copolymer (B) is 20% by mass or less, changes in mechanical properties can be suppressed.

### <Anti-blocking particle (Q)>

The resin composition of the present invention may contain an anti-blocking particle (Q) as necessary.

The anti-blocking particle (Q) is used to impart anti-blocking properties to the raw materials used in the resin composition of the present invention, the resin composition of the present invention, the molding material made of the resin composition of the present invention, and the molded article thereof. In particular, it is preferable to use it to prevent blocking of pellets, beads, powder, and the like.

Among them, the anti-blocking particle (Q) can be used for the purpose of preventing blocking of the particles of the (meth)acrylic-based copolymer (B). That is, it is preferable to add the anti-blocking particle (Q) when the (meth)acrylic-based copolymer (B) is produced by suspension polymerization and collected as beads. The timing of adding the anti-blocking particle (Q) may be such that it is added in the state of a polymer slurry obtained by the suspension polymerization, or in the step of dewatering the polymer slurry, or in the step of recovering the beads after dewatering.

The anti-blocking particle (Q) is adsorbed on the surface of the (meth)acrylic-based copolymer (B) and has the effect of preventing blocking.

The ratio of the anti-blocking particles(Q) relative to 100 parts by mass of the (meth)acrylic-based copolymer (B) is preferably 0.01 parts by mass or more, more preferably 0.05 parts by mass or more, and even more preferably 0.1 parts by mass or more. Furthermore, the ratio of the anti-blocking particle (Q) relative to 100 parts by mass of the (meth)acrylic-based copolymer (B) is preferably 50 parts by mass or less, more preferably 30 parts by mass or less, and even more preferably 20 parts by mass or less. When the ratio of the anti-blocking particles (Q) is 0.01 parts by mass or more, the powder has excellent fluidity and is easy to handle. When the ratio of the anti-blocking particles (Q) is 50 parts by mass or less, the performance of the (meth)acrylic-based copolymer (B) is not impaired.

The particle size of the anti-blocking particle (Q) is preferably 35% or less, more preferably 20% or less, even more preferably 10% or less, and particularly preferably 5% or less of the median diameter of the (meth)acrylic-based copolymer (B) measured using a particle size distribution meter. The particle size of the blocking prevention particle (Q) is preferably 0.1% or more, more preferably 0.2% or more, even more preferably 0.3% or more, and particularly preferably 0.5% or more of the median diameter of the (meth)acrylic-based copolymer (B) measured using a particle size distribution meter. When the particle size of the anti-blocking particle (Q) is 35% or less and 0.1% or more of the median diameter of the (meth)acrylic-based copolymer (B) measured using a particle size distribution meter, the powder has excellent fluidity and is easy to handle. The median diameter refers to the center value when the particle size data are arranged in order from the minimum value to the maximum value.

Examples of the anti-blocking particle (Q) include a powder of a (meth)acrylic-based polymer different from the (meth)acrylic-based copolymer (B), a particle made of a vinyl chloride polymer, and an inorganic fine particle such as silica. Among these, the powder of a (meth)acrylic-based polymer different from the (meth)acrylic-based copolymer (B) is preferably used.

### <Method of producing the resin composition>

The method of producing the resin composition of the present invention by mixing the thermoplastic polyurethane (A1), the (meth)acrylic-based copolymer (B), and other components used as necessary is not particularly limited, and examples include a physical mixing method using a Henschel mixer, blender, or the like, a melt kneading method using a Brabender, kneader, extruder, or the like, and a blend casting method in which the resin composition is dissolved in a solvent.

### [Molding material]

The resin composition of the present invention can be used as a molding material. As a molding method for the resin composition of the present invention, in addition to extrusion molding using a kneading extruder such as a single-screw extruder, a twin-screw extruder, or the like, it can also be applied to ordinary known molding methods such as injection molding, blow molding, and roll processing to obtain various desired molded articles.

There is no particular limitations on the shape of the molding material, however when it is assumed that the molding material will be used to melt-mold a molded article, it is preferable that the thermoplastic polyurethane (A1), the (meth)acrylic-based copolymer (B), and other components to be blended as necessary are melt kneaded in advance and processed them into pellets or beads.

The content of the (meth)acrylic-based copolymer (B) relative to the total mass of the molding material is preferably 1% by mass or more, more preferably 5% by mass or more, and even more preferably 10% by mass or more. The content of the (meth)acrylic-based copolymer (B) relative to the total mass of the molding material is preferably 50% by mass or less, more preferably 30% by mass or less, and even more preferably 20% by mass or less. When the content of the (meth)acrylic-based copolymer (B) is 1% by mass or more and 50% by mass or less, the protein adhesion suppression ability and moldability are excellent.

From the viewpoint of transparency of the obtained molded article, the HAZE value measured with reference to ISO14782 for a test piece having a thickness of 2 mm obtained by molding the resin composition of the present invention is preferably 0% or more and 50% or less, more preferably 0% or more and 30% or less, and even more preferably 0% or more and 25% or less. When the HAZE value is 50% or less, sufficient transparency is obtained.

### [Uses]

The resin composition of the present invention is suitable as a molding material.

As shown in the Examples below, the resin composition of the present invention has excellent transparency and has a protein adhesion suppression ability, so that the resin composition of the present invention is suitable for producing an article (molded article) that comes into contact with proteins. It is particularly suitable for producing an article that come into contact with plasma proteins.

The molding material of the present invention has protein adhesion suppression ability. In the protein adhesion test described below, the molding material of the present invention can achieve a fibrinogen adsorption amount of less than 1.25 µg (micrograms) per 1 cm². The fibrinogen adsorption amount is preferably 1.20 µg/cm² or less, and more preferably 1.10 µg/cm² or less.

### <Protein adhesion test: µBCA method>

The molding material to be tested is immersed in a 1 mg/mL fibrinogen (Fib) solution dissolved in phosphate buffered saline (PBS) at 37°C for 2 hours. After immersion for 2 hours, the molding material is washed with PBS, and then immersed in 6 mL of an aqueous solution of sodium dodecyl sulfate and subjected to ultrasonic cleaning for 5 minutes. 150 µL of the solution after ultrasonic cleaning is placed in a 96-well plate, and 150 µL of a protein quantification reagent from a commercially available BCA kit is placed in the portion containing the solution after ultrasonic cleaning, and the plate is kept at 37°C for 2 hours. After keeping for 2 hours, the absorbance at 562 nm is measured using a plate reader, and the amount of adsorbed Fib is calculated by applying the result to a calibration curve obtained from a Fib solution of known concentration.

### [Molded article]

Examples of the shape of the article (molded article) obtained by molding the molding material of the present invention include a sheet, a film, a tube, a packing, or a three-dimensional shape. Furthermore, the surface of the molded article may be a mirror surface, or one or both sides may be given a surface unevenness treatment such as an emboss, matte finish, or the like. A protective film or separator may be placed on the surface of the molded article. Various powders may be attached to the surface to prevent blocking.

### <Method of manufacturing molded article>

The method of manufacturing an article (molded article) by molding the molding material of the present invention is preferably a melt molding method. Examples of melt molding methods include an injection molding method, a compression molding method, a blow molding method, an extrusion molding method, a rotational molding method, a casting method, and a solvent casting method. Among these, an injection molding method and an extrusion molding method are preferred from the viewpoint of productivity. There are no particular limitations on the molding die, resin temperature, molding conditions, and the like, used in molding by the above molding methods.

When manufacturing a molded article, it is preferable to pre-dry the molding material at an appropriate temperature and time in order to set the moisture content of the molding material to an appropriate range, just as in the case of producing the molding material described above. When the pre-drying temperature is low or the pre-drying time is short, problems such as foaming and poor appearance occur due to melt molding of a molding material containing volatile components. Conversely, when the pre-drying temperature is too high or the pre-drying time is too long, the molding material may be thermally deteriorated, resulting a decrease in the physical properties and coloring of the molded article.

### <Processing using the molded article>

The molded article can be bonded or welded to other parts, or sheets, films, or tubes can be bonded or fused together to produce various products. Examples of methods for welding the molded article using the resin composition of the present invention, i.e., the molding material of the present invention, include ultrasonic welding, vibration welding, high-frequency welding, hot plate welding, laser welding, spin welding, and the like. In the method for bonding the molded article, various adhesives such as epoxy resin-based, vinyl acetate-based, acrylic resin-based, phenolic resin-based, chloroprene rubber-based, nitrile rubber-based, silicone rubber-based, styrene butadiene rubber-based, cyanoacrylate-based adhesives, and the like can be appropriately selected and used. Solvent bonding can also be applied. Furthermore, tape bonding using tape attachment or double-sided tape can also be used.

When bonding different materials together, multiple bonding methods can be combined depending on the materials to be bonded.

The article (molded article) is preferably an article that comes into contact with body fluids containing proteins (blood, digestive fluids, exudates, and the like), and examples of such articles include medical device parts such as a coating, a sealant, a packing, a tube, and the like.

For example, the article is suitable for medical device parts such as a catheter, a guide wire, an endoscope, a syringe, a drip route, a cannula, gauze, a stent, a shunt tube, and a drain.

The article is also suitable for cardiovascular catheters including a central venous catheter, a peripheral venous catheter, a coronary artery catheter, and a CV port catheter, a blood collection kit, a medication tube, a regenerative medicine-related tube, and the like, which require both protein adhesion suppression ability and transparency.

### [Examples]

The present invention will be explained in more detail below with reference to Examples and Comparative Examples. The present invention is not limited to these Examples.

In the following, "parts" means "parts by mass".

### [Molecular weight]

Mw and Mn were determined using gel permeation chromatography (GPC). The measurement conditions are as follow.
Apparatus: HLC-8220 (manufactured by Tosoh Corporation)
Column: TSK GUARD COLUMN SUPER H-H (4.6 x 35 mm, manufactured by Tosoh Corporation) and two TSK-GEL SUPER HM-Hs (6.0 x 150 mm, manufactured by Tosoh Corporation) connected in series
Eluent: Tetrahydrofuran
Measurement temperature: 40°C
Flow rate: 0.6 mL/min

The Mw (mass average molecular weight) and Mn (number average molecular weight) were calculated using a calibration curve created using polymethyl methacrylate (four types: Mp (peak molecular weight) = 141,500, 55,600, 11,100, and 1,590) manufactured by Polymer Laboratories.

### [Refractive index]

The refractive index was measured under the following conditions in accordance with JIS K7142 (Method A) using the following equipment.
- Instrument name: Multi-wavelength Abbe refractometer DR-M4 (manufactured by Atago Co., Ltd.)
- Sample preparation: Measurements were performed using sheets obtained by heat pressing or injection molding.
- Intermediate liquid: 1-bromonaphthalene

### [Transparency]

The HAZE value of each of the molded plates having a thickness of 2 mm obtained by melt-molding the resin compositions according to the methods of the Examples and Comparative Examples was measured in accordance with ISO 14782 using the following measuring equipment.
- Measuring equipment: NDH2000 (manufactured by Nippon Denshoku Industries Co., Ltd.)

It can be evaluated that the smaller the HAZE value, the more excellent the transparency.

### [Protein adhesion test (µBCA method)]

Each of the molded plates having a thickness of 2 mm obtained by melt molding in the Examples and Comparative Examples was cut into a size of 10 mm x 40 mm and immersed in a 1 mg/mL fibrinogen (Fib) solution dissolved in phosphate buffered saline (PBS) at 37°C for 2 hours. After immersion for 2 hours, the molded plate was washed with PBS, and then immersed in 6 mL of an aqueous solution of sodium dodecyl sulfate and subjected to ultrasonic cleaning for 5 minutes. 150 µL of the solution after ultrasonic cleaning was placed in a 96-well plate, and 150 µL of a protein quantification reagent from a commercially available BCA kit was placed in the portion containing the solution after ultrasonic cleaning, and the plate was kept at 37°C for 2 hours. After keeping for 2 hours, the absorbance at 562 nm was measured using a plate reader, and the amount of adsorbed Fib was calculated by applying the result to a calibration curve obtained from a Fib solution of known concentration.

It can be evaluated that the smaller the amount of Fib adsorption, the better the protein adhesion suppression ability.

### [Tensile strength/Elongation at break]

The resin composition was subjected to the heat pressing or injection molding as described in the Examples to obtain a sheet having a thickness of 2 mm, which was then annealed at 80°C for 15 hours and then punched out with a JIS No. 3 dumbbell to prepare a test specimen.

The test conditions were a tensile speed of 200 mm/min in accordance with ISO 37, and measurements were carried out using an Autograph AG-X (manufactured by Shimadzu Corporation).

### [Thermoplastic polyurethane]

TPU1: ChronoFlex (registered trademark) AL75A manufactured by Mitsubishi Chemical Corporation
(isocyanate: aliphatic, polyol: polycarbonate-based)
TPU2: ChronoFlex (registered trademark) AL85A manufactured by Mitsubishi Chemical Corporation
(isocyanate: aliphatic, polyol: polycarbonate-based)
TPU3: ChronoFlex (registered trademark) AL93A manufactured by Mitsubishi Chemical Corporation
(isocyanate: aliphatic, polyol: polycarbonate-based)
TPU4: ChronoThane (registered trademark) T85A manufactured by Mitsubishi Chemical Corporation
(isocyanate: aliphatic, polyol: polyether-based)
TPU5: ChronoFlex (registered trademark) AL65D manufactured by Mitsubishi Chemical Corporation
(isocyanate: aliphatic, polyol: polycarbonate-based)
TPU6: Pandex (registered trademark) T9290 manufactured by DIC Covestro Polymer Ltd.
(isocyanate: aromatic, polyol: polycarbonate-based)
TPU7: ChronoFlex (registered trademark) AL80A manufactured by Mitsubishi Chemical Corporation
(isocyanate: aliphatic, polyol: polycarbonate-based)

### [(Meth)acrylic-based copolymer (B)]

### <Production Example 1: Synthesis of dispersant (1)>

In a reaction apparatus equipped with a stirrer, a cooling tube, and a thermometer, 61.6 parts of a 17% by mass aqueous solution of potassium hydroxide, 19.1 parts of methyl methacrylate, and 19.3 parts of deionized water were charged. The liquid in the reaction apparatus was then stirred at room temperature, and after confirming the exothermic peak, the mixture was stirred for 4 hours. The reaction liquid in the reaction apparatus was then cooled to room temperature to obtain an aqueous solution of potassium methacrylate.

Next, 900 parts of deionized water, 70 parts of a 42 mass% aqueous solution of sodium 2-sulfoethyl methacrylate (manufactured by Mitsubishi Chemical Corporation, product name: ACRYESTER (registered trademark) SEM-Na), 16 parts of the above aqueous solution of potassium methacrylate, and 7 parts of methyl methacrylate were added in a reaction apparatus equipped with a stirrer, a cooling tube, and a thermometer, and the mixture was stirred while replacing the atmosphere inside the reaction apparatus with nitrogen, and the liquid in the reaction apparatus was heated to 50°C. Into the reactor, 0.053 parts of V-50 (manufactured by Fujifilm Wako Pure Chemical Corporation, 2,2'-azobis(2-methylpropionamidine) dihydrochloride, product name) was added as a polymerization initiator, and the liquid in the reaction apparatus was heated to 60°C. After the polymerization initiator was added, 1.4 parts of methyl methacrylate was added in portions every 15 minutes for a total of 5 times (total amount of methyl methacrylate: 7 parts). After this, the liquid in the reaction apparatus was kept at 60°C for 6 hours while stirring, and then cooled to room temperature to obtain a dispersant (1) having a solid content of 10% by mass as a transparent aqueous solution.

### <Production Example 2: Synthesis of chain transfer agent (1)>

In a synthesis apparatus equipped with a stirrer, 2.00 g (8.03 mmol) of cobalt (II) acetate tetrahydrate (manufactured by Fujifilm Wako Pure Chemical Corporation, special grade), 3.86 g (16.1 mmol) of diphenylglyoxime (manufactured by Tokyo Chemical Industry Co., Ltd., EP grade), and 100 mL of diethyl ether that had been deoxygenated by nitrogen bubbling were added under a nitrogen atmosphere and the mixture was stirred at room temperature for 2 hours.

Next, 20 mL of boron trifluoride diethyl ether complex (manufactured by Tokyo Chemical Industry Co., Ltd., EP grade) was added and stirred for another 6 hours. The resulting product was filtered, the solid was washed with diethyl ether, and dried at 100 MPa or less at 20°C for 12 hours to obtain 5.02 g (7.93 mmol, yield 99% by mass) of a chain transfer agent (1) as a reddish brown solid.

### <Production Example 3: Synthesis of macromonomer (d'-1)>

In a polymerization apparatus equipped with a stirrer, a cooling tube, and a thermometer, 135 parts of deionized water, 0.1 parts of sodium sulfate (Na₂SO₄), and 0.26 parts of the dispersant (1) (solid content 10% by mass) produced in Production Example 1 were added and stirred to obtain a uniform aqueous solution. Next, 95 parts of methyl methacrylate, 5 parts of methyl acrylate, 0.0010 parts of the chain transfer agent (1) produced in Production Example 2, and 0.1 parts of Perocta (registered trademark) O (1,1,3,3-tetramethylbutylperoxy 2-ethylhexanoate, product name, manufactured by NOF Corporation) as a polymerization initiator were added to obtain an aqueous dispersion.

Then, the inside of the polymerization apparatus was thoroughly replaced with nitrogen, and the aqueous dispersion was heated to 80°C and maintained at that temperature for 3 hours, and then heated to 90°C and maintained at that temperature for 2 hours. The reaction liquid was then cooled to 40°C to obtain an aqueous suspension of the macromonomer. This aqueous suspension was filtered through a filter cloth, the filtrated residue was washed with deionized water, and dried at 40°C for 16 hours to obtain a macromonomer (d'-1). The macromonomer (d'-1) had an Mw of 36,000 and an Mn of 15,000.

### <Production Example 4: (Meth)acrylic copolymer (B-1)>

In a reaction apparatus equipped with a stirrer, a cooling tube, and a thermometer, 145 parts of deionized water, 0.36 parts of sodium sulfate, 1.25 parts of the dispersant (1) produced in Production Example 1, 40 parts of the macromonomer (d'-1) produced in Production Example 3, 60 parts of methoxyethyl acrylate (manufactured by Osaka Organic Chemical Industry Ltd.), and 0.2 parts of n-octyl mercaptan (manufactured by Kanto Chemical Co., Inc., product name) were charged, and the mixture was heated to 55°C with stirring to obtain a composition in a syrup dispersion state. After cooling the composition to 40°C or lower, 0.12 parts of V601 (manufactured by Fujifilm Wako Pure Chemical Corporation, product name: dimethyl 2,2'-azobis(2-methylpropionate)) was dissolved in the composition to obtain a polymerizable composition in a syrup dispersion state.

Then, the polymerizable composition in the syrup dispersion state was heated to 75°C and maintained at that temperature for 2 hours. After that, the temperature was raised to 85°C and maintained at that temperature for 90 minutes. Then, the suspension was cooled to 40°C or lower and then filtered. The resulting filtrated residue was washed with deionized water and dried at 70°C for 12 hours to obtain a (meth)acrylic copolymer (B-1). The obtained (meth)acrylic copolymer (B-1) had an Mn of 56,000 and an Mw of 300,000. The median diameter of the obtained (meth)acrylic copolymer (B-1) was 560 µm, as measured using a particle size distribution meter (manufactured by Horiba, Ltd., LA-960).

The refractive index of the (meth)acrylic copolymer (B-1) was 1.485.

### [Example 1]

85 parts of the thermoplastic polyurethane (TPU1) and 15 parts of the (meth)acrylic copolymer (B-1) obtained in Production Example 4 were dry blended and injected into a microcompounder manufactured by Rheo Lab Co., Ltd., then mixture was plasticized at a cylinder temperature of 190 to 210°C and a screw rotation speed of 100 rpm to obtain an injection molded plate.

The obtained molded plate was evaluated for transparency, tensile strength, and elongation at break. The evaluation results are shown in Table 1.

### [Examples 2-4, Comparative Examples 1 and 2]

Except for changing the thermoplastic polyurethane (A1) to the type shown in Table 1, the evaluations were performed in the same manner as in Example 1. The evaluation results are shown in Table 1.

Table 1 also shows the refractive index and the difference in the refractive index between the thermoplastic polyurethane (A1) and the (meth)acrylic-based copolymer (B) used in each Example.

**[Table 1]**

| | | | Example 1 | Example 2 | Example 3 | Example 4 | Comparative Example 1 | Comparative Example 2 |
|---|---|---|---|---|---|---|---|---|
| Thermoplastic polyurethane (A1) | | | T P U 1 | T P U 2 | T P U 3 | T P U 4 | T P U 5 | T P U 6 |
| Isocyanate | | | Aliphatic | Aliphatic | Aliphatic | Aliphatic | Aliphatic | Aromatic |
| Polyol | | | Polycarbonate-based | Polycarbonate-based | Polycarbonate-based | Polyether-based | Polycarbonate-based | Polycarbonate-based |
| Refractive index | Thermoplastic polyurethane (A1) | | 1.490 | 1.493 | 1.497 | 1.499 | 1.504 | 1.537 |
| | (Meth)acrylic-based copolymer (B-1) | | 1.485 | 1.485 | 1.485 | 1.485 | 1.485 | 1.485 |
| Refractive index difference | | | 0.005 | 0.008 | 0.012 | 0.014 | 0.019 | 0.052 |
| Haze | | % | 20 | 19 | 19 | 2 | 78 | 99 |
| Tensile strength | | [MPa] | 38 | 39 | 35 | 15 | 29 | 26 |
| Elongation at break | | [%] | 360 | 350 | 290 | 508 | 320 | 420 |

As shown in Comparative Examples 1 and 2 in Table 1, when the difference in the refractive index between the thermoplastic polyurethane (A1) and the (meth)acrylic-based copolymer (B) exceeds 0.018, transparency becomes insufficient.

### [Examples 5 to 9, Comparative Example 3]

The thermoplastic polyurethane (TPU7) and the (meth)acrylic-based copolymer (B-1) obtained in Production Example 4 were melt-kneaded at 170 to 190°C using KZW15-45MG-NH manufactured by Technovel Corporation to obtain a resin composition.

For the protein adhesion test, the obtained resin composition was injected into a microcompounder manufactured by Rheo Lab Co., Ltd. and then plasticized at a cylinder temperature of 190 to 210°C and a screw rotation speed of 100 rpm to obtain an injection molded plate.

For the tensile test, the resin composition was heated at 190°C for about 6 minutes using a Shinto F hydraulic press manufactured by Shinto Metal Industries, Ltd., and then pressurized at 5 MPa for 3 minutes while keeping the temperature constant, and cooled and solidified at normal temperature and 10 MPa to obtain a sheet.

The results of the protein adhesion test and the measurement results of the tensile strength and elongation at break are shown in Table 2.

**[Table 2]**

| | | Comparative Example 3 | Example 5 | Example 6 | Example 7 | Example 8 | Example 9 |
|---|---|---|---|---|---|---|---|
| Thermoplastic polyurethane (A1) | % by mass | 100 | 85 | 70 | 60 | 55 | 50 |
| (Meth)acrylic-based copolymer (B-1) | % by mass | 0 | 15 | 30 | 40 | 45 | 50 |
| Protein adhesion test (µBCA method) | Fib adsorption amout (µg/cm²) | 1.27 | 0.68 | 0.20 | 0.27 | 0.29 | 0.20 |
| Tensile strength | [MPa] | 48.2 | 48.6 | 30.1 | 7.7 | 7.1 | 6.8 |
| Elongation at break | % | 450 | 450 | 420 | 280 | 260 | 230 |

Table 2 shows that the content of the (meth)acrylic-based copolymer (B) in the resin composition is preferably 10% by mass or more from the viewpoint of protein adhesion suppression ability, and less than 50% by mass from the viewpoint of tensile strength.

### Industrial Applicability

The resin composition of the present invention can impart a protein adhesion suppression ability to the obtained molded article.

The resin composition of the present invention is suitable for articles that come into contact with protein containing body fluids (blood, digestive fluids, exudates, and the like).

Examples of such articles include medical device parts such as a tube, a coating, a sealing, and a packing. More specifically, the present invention is suitable for medical device parts such as a catheter, a guide wire, an endoscope, a syringe, a drip route, a cannula, gauze, a stent, a shunt tube, and a drain.

Although the present invention has been described in detail with reference to particular embodiments, it will be apparent to those skilled in the art that various changes may be made thereto without departing from the spirit and scope of the present invention.

The present application is based on Japanese Patent Application No. 2022-180712 filed on November 11, 2022, which is herein incorporated in its entirety by reference.

## Claims

1. A resin composition comprising:
a thermoplastic polyurethane (A1); and
a (meth)acrylic-based copolymer (B),
wherein the (meth)acrylic-based copolymer (B) is an amphiphilic copolymer, and
a difference in a refractive index between the thermoplastic polyurethane (A1) and the (meth)acrylic-based copolymer (B) is 0.018 or less.

2. The resin composition according to claim 1, wherein the refractive index of the thermoplastic polyurethane (A1) is 1.490 or more and 1.503 or less.

3. The resin composition according to claim 1 or 2, wherein the refractive index of the (meth)acrylic-based copolymer (B) is 1.472 or more and 1.521 or less.

4. A resin composition comprising:
a thermoplastic polyurethane (A2); and
a (meth)acrylic-based copolymer (B),
wherein the thermoplastic polyurethane (A2) comprises a structural unit derived from at least one of an aliphatic isocyanate compound and an alicyclic isocyanate compound, and
the (meth)acrylic-based copolymer (B) is an amphiphilic copolymer.

5. The resin composition according to claim 1, wherein the thermoplastic polyurethane (A1) comprises a structural unit derived from at least one of an aliphatic isocyanate compound and an alicyclic isocyanate compound.

6. The resin composition according to claim 5, wherein the thermoplastic polyurethane (A1) comprises a hard segment comprising a structural unit derived from at least one of the aliphatic isocyanate compound and the alicyclic isocyanate compound and a chain extender, and a soft segment comprising a structural unit derived from a polyol.

7. The resin composition according to claim 6, wherein a content of the hard segment is 20% by mass or more and 65% by mass or less based on 100% by mass of the thermoplastic polyurethane (A1).

8. The resin composition according to claim 7, wherein the content of the hard segment is 25% by mass or more and 50% by mass or less based on 100% by mass of the thermoplastic polyurethane (A1).

9. The resin composition according to claim 1 or 4, wherein a content of the (meth)acrylic-based copolymer (B) is 1 part by mass or more and 50 parts by mass or less based on 100 parts by mass of the resin composition.

10. The resin composition according to claim 1 or 4, wherein the (meth)acrylic-based copolymer (B) is an amphiphilic copolymer having a polymer chain (B1) of a monomer unit (b1) represented by the following formula (B1). 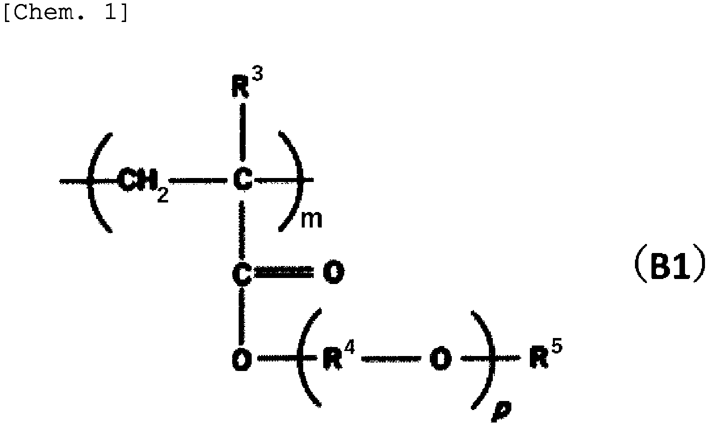 (In the formula (B1), R³ represents a hydrogen atom or a methyl group. R⁴ represents an alkylene group having 1 to 4 carbon atoms. R⁵ represents a hydrocarbon group having 1 to 6 carbon atoms. p is a natural number from 1 to 10. m is a natural number from 2 to 1,000,000.)

11. The resin composition according to claim 10, wherein the monomer unit (b1) is a monomer unit derived from at least one monomer (b'1) selected from the group consisting of methoxymethyl acrylate, methoxyethyl acrylate, methoxypropyl acrylate, methoxybutyl acrylate, methoxypolyethylene glycol acrylate, methoxymethyl methacrylate, methoxyethyl methacrylate, methoxypropyl methacrylate, methoxybutyl methacrylate, and methoxypolyethylene glycol methacrylate.

12. The resin composition according to claim 10, wherein the (meth)acrylic-based copolymer (B) is an amphiphilic copolymer having a constituent unit derived from a monomer represented by the following formula (B2). 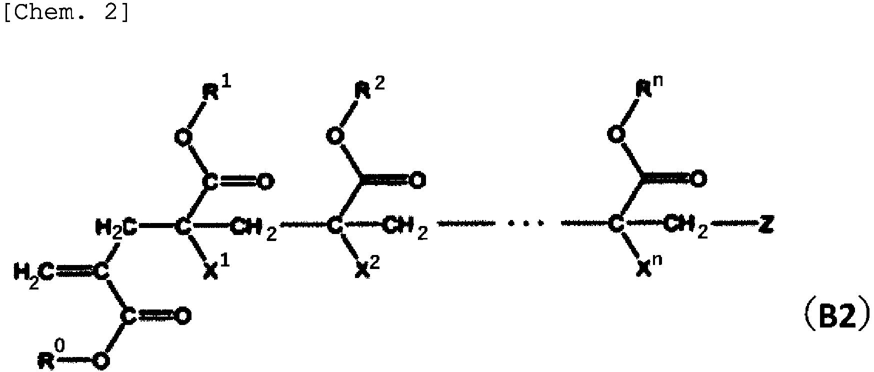 (In the formula (B2), R⁰ to Rⁿ each independently represent a hydrogen atom, an unsubstituted or substituted alkyl group, an unsubstituted or substituted alicyclic group, an unsubstituted or substituted aryl group, an unsubstituted or substituted heteroaryl group, or an unsubstituted or substituted non-aromatic heterocyclic group. Multiple R⁰ to Rⁿ may be the same or different from each other. X¹ to Xⁿ represent a hydrogen atom or a methyl group. Multiple X¹ to Xⁿ may be the same or different from each other. Z is a terminal group. n is a natural number from 2 to 10,000.)

13. The resin composition according to claim 1 or 4, wherein the (meth)acrylic-based copolymer (B) has a weight average molecular weight (Mw) of 75,000 or more.

14. A molding material comprising the resin composition according to claim 1 or 4.

15. A medical device part comprising the resin composition according to claim 1 or 4.

16. The medical device part according to claim 15, wherein the medical device part is for in contact with plasma proteins.

17. The medical device part according to claim 15, wherein the medical device part is a tube.

18. The medical device part according to claim 15, wherein the medical device part is a coating.

19. The medical device part according to claim 15, wherein the medical device part is a sealing.

20. The medical device part according to claim 15, wherein the medical device part is a packing.

21. The medical device part according to claim 15, wherein the medical device part is a catheter.
